# EUROPEAN PATENT SPECIFICATION

(11) **EP 0 593 136 B1**
(45) Date of publication and mention of the grant of the patent: **05.03.1997**
(21) Application number: 93203354.1
(22) Date of filing: 11.12.1990
(51) Int. Cl.: A61N 5/10

(54) **Device for the prevention of arterial restenosis**
Einrichtung zum Verhindern von arterieller Restenose
Dispositif pour la prévention de la resténose artérielle

(30) Priority: 11.12.1989 US 448691
(43) Date of publication of application: 20.04.1994
(62) Divisional of application: 90313433.6
(73) Proprietor: Fischell, Robert E., Dayton, Maryland 21036 (US); FISCHELL, Tim A., Los Altos, CA 94022 (US)
(72) Inventor: Fischell, Robert E., Dayton, Maryland 21036 (US); FISCHELL, Tim A., Los Altos, CA 94022 (US)
(74) Representative: Harris, Ian Richard

(56) References cited:
- GB-A- 857 992
- US-A- 2 546 761
- US-A- 3 811 426
- US-A- 4 096 862
- US-A- 4 819 618

## Description

This invention relates to devices for intra-arterial insertion.

Since the mid-to late 1980's, intra-arterial stents have found extensive use as a treatment to prevent restenosis subsequent to balloon angioplasty or atherectomy. A recurrent problem is that excessive tissue growth (intimal hyperplasia) at the site of the balloon dilation or atherectomy plaque excision results in restenosis of the artery. One possible solution to this problem (US Patent No. 4,768,507) is to coat the stent with an anti-thrombogenic surface so as to reduce platelet fibrin deposition. Although an anti-thrombogenic coating can prevent acute thrombotic arterial closure and decrease the need for anticoagulant drug therapy, there is still an urgent need to decrease restenosis which is caused by intimal hyperplasia.

It is well known that radiation therapy can reduce the proliferation of rapidly growing cancer cells in a malignant tumour, and this is made use in the present invention, which resides in a stent comprising a tubular structure insertable into an artery and locatable therein to maintain the lumen of the artery patent, wherein the stent comprises or is constructed of a material that is radioactive. Preferably the stent comprises a helical wire spring of the type shown in US Patent 4,768,507 and which either incorporates or is coated with a radioactive isotope, preferably a beta emitter.

Tubular radioactive structures for insertion into the body are, of course, known. For example, US-A-3,351,049 discloses a radioactive seed for radiation therapy comprising an elongate stainless steel tube, sealed at both ends and having sealed therein a radioactive source, e.g. a filamentous nylon body impregnated with a soft X-ray emitter such as I¹²⁵. Such seeds are simply used as a radioactive implant to be implanted in a tumour to provide a highly localised source of radioactivity.

An even more basic radioactive seed is disclosed in US-A-1954868 dating from 1929. This simply consists of a single wall capillary tube sealed at both ends and containing a quantity of radon as the radioactive source. A similar seed is shown in DE-C-867433. In this case the seed comprises a tube of a radioactive isotope sealed in a carrier tube, e.g. of Monel metal, glass, ceramic or graphite. Such devices are of little relevance to the object of the present invention, i.e. the prevention of restenosis in an artery following balloon angioplasty or atherectomy.

US patent 4,819,618 describes an iridium/platinum implant which comprises an iridium 192 core having an outer platinum sheath. The implant thus radiates gamma radiation. The implant can be attached to the end of a wire for insertion in a tumor.

US patent 3,811,426 describes a method and apparatus for in-vessel radiation treatment of blood by supporting a radioactive isotope in the middle of a wire suspended across a blood vessel and proposes the use of beta particle emitters for this purpose.

In the parent application (EP-A-04433011) from which the present application is divided, an intra-arterial stent is disclosed for use in the prevention of restenosis of an artery following balloon angioplasty or atherectomy and resulting from intimal hyperplasia, that stent consisting essentially of an expansible radioactive tubular structure, preferably a helical wire coil, that is insertable into the artery in a collapsed condition and which is expansible therein to bring the radioactive stent into contact with the inner surface of the artery.

In accordance with the present invention, there is provided a thin wire comprising a radioactive tip for use in the prevention of restenosis of an artery following arterial trauma, said thin wire being configured so that said radioactive tip can be inserted temporarily at the site of an arterial wall trauma and then be withdrawn and serves when so inserted to prevent restenosis of the artery at the trauma site, said radioactive tip including a beta-particle emitter radioisotope and emitting beta-particles.

The accompanying drawings refer to the stent of the parent application and not to the present invention.
Figure 1 is a cross-section showing two turns of the radioactive helical coil spring stent embedded into a balloon dilated or atherectomized plaque within a human artery;
Figure 2 is a cross-section through the spring wire of the radioactive helical coil spring stent according to the parent application showing a radioisotope core material within the spring material;
Figure 3 is a cross-section through the spring wire of a helical coil spring stent according to the parent application showing a thin plating or radioisotope material on the exterior surface;
Figure 4 is a cross-section through a central core spring wire of a helical coil spring stent according to the parent application showing a radioisotope plating which is covered with an anti-thrombogenic coating.

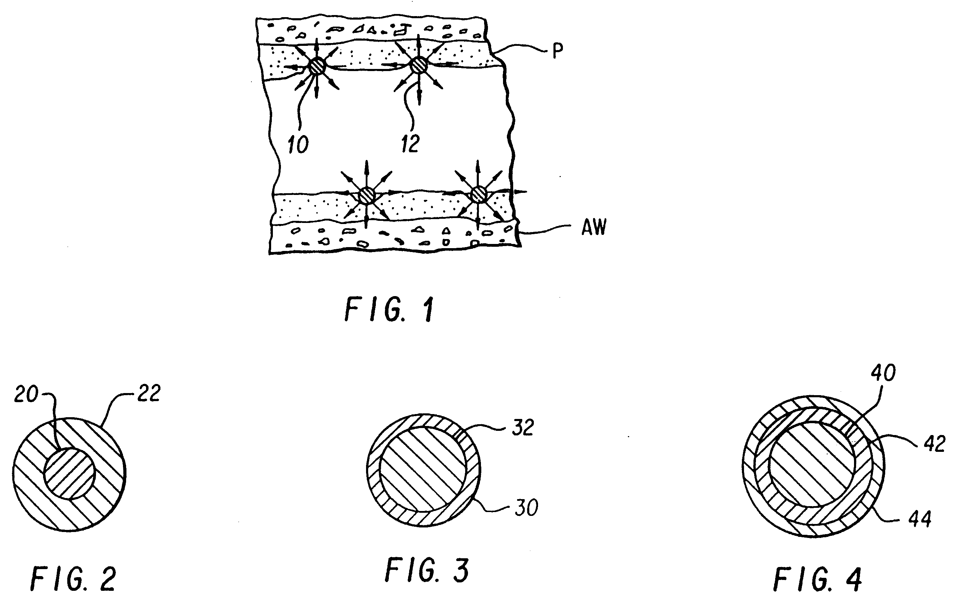

Referring to the drawings, the stent comprises a radioactive helical coil spring (10) fabricated from a pure metal or alloy and coated with or incorporating a radioactive isotope and which in use is imbedded into plaque (P) within the arterial wall of the artery (AW) as is shown in Figure 1. In situ, the radioactive stent emits radiation in the direction of the arrows (12) pointing outward from the wire (10) which indicate the omnidirectional emission of particles from the stent wire. The purpose of this radiation is to decrease the rate of proliferative cell growth of the traumatized arterial wall AW (which growth is termed "intimal hyperplasia"). Thus it would be expected that restenosis, which frequently occurs after stent implantation, will be significantly reduced.

The radioisotope used for that purpose is a beta-particle emitting radioisotope in that predominantly beta particles are emitted, although some alpha and gamma particles could also be emitted. The half-life would ideally be between 10 hours and 100 days. An optimum emitter might be a beta emitting isotope such as vanadium 48 which has a half-life of 16 days and only 8% of its emitted energy is from gamma radiation. The ideal attribute of a beta emitter is that the radiation does not travel very far in human tissue. Thus only the tissue in close proximity to the radioisotope stent will be affected. Furthermore, only moderate levels of radiation are desired since it is known that very high levels can cause injury to non-proliferating tissues.

Another method to make the material of the stent spring wire is from a metal into which is alloyed an element that can be made into a radioisotope. For example, phosphorus 32, a 14.3 day half-life beta emitter, could be alloyed into steel which could be used for the stent wire.

Figure 2 shows a stent wire cross-section in which a wire made from a radioisotope core material 20 is formed within an outer covering 22 that has the attributes that are desirable for being a coil spring stent.

Figure 3 shows a cross-section of an alternative embodiment in which a radioisotope coating 30 is plated onto a spring material core 32. For example, the beta emitting isotope gold 198 (half-life 2.7 days) could be used to coat any suitable spring metal material.

Figure 4 shows a more complex stent cross-section in which a core of some material ideally suited for stents is plated with a radioisotope coating 42 which is, in turn, coated with an anti-thrombogenic coating 42 such as carbon as described in US Patent No. 4,768,507.

Although helical coil spring stents have generally been described in the parent application the concept of utilizing a radioactive material within a stent structure so as to attenuate intimal hyperplasia is certainly applicable to other devices. In particular, and in accordance with the present invention, that concept is extended to a thin wire with a radioactive tip which can temporarily be placed at the site of the vessel wall trauma to prevent intimal hyperplasia and consequent restenosis of the artery, that wire being withdrawn after a limited time. Other than being a thin wire with a radioactive tip, the principle and the materials used are the same.

## Claims

1. A thin wire comprising a radioactive tip for use in the prevention of restenosis of an artery following arterial trauma, said thin wire being configured so that said radioactive tip can be inserted temporarily at the site of an arterial wall trauma and then be withdrawn and serves when so inserted to prevent restenosis of the artery at the trauma site, said radioactive tip including a beta-particle emitter radioisotope and emitting beta-particles.

2. A thin wire according to Claim 1, wherein the wire comprises said beta-particle emitter radioisotope incorporated into the material of the wire.

3. A thin wire according to Claim 1, wherein the wire comprises said beta-particle emitter radioisotope plated onto the surface of the wire.

4. A thin wire according to any one of Claims 1 to 3, wherein said beta-particle radioisotope has a half-life of less than 100 days.

5. A thin wire according to Claim 4, wherein said beta-particle emitter radioisotope is vanadium 48 or gold 198.

6. A thin wire according to any one of Claims 1 to 5, wherein said wire is coated with anti-thrombogenic material.

7. A thin wire according to any one of claims 1 to 3, wherein said beta-particle emitter radioisotope is phosphorous 32.

## Patentansprüche

1. Dünner Draht mit einer radioaktiven Spitze für die Verwendung zur Verhinderung von Restenose einer Arterie nach arteriellem Trauma, wobei dieser dünne Draht so gestaltet ist, daß die radioktive Spitze zeitweilig an der Stelle eines arteriellen Wandtraumas eingefügt und dann herausgezogen werden kann, und so eingefügt dazu dient, Restenose der Arterie an der Traumastelle zu verhindern, wobei die radioaktive Spitze ein beta-Teilchenemitterradioisotop einschließt und beta-Teilchen emittiert.

2. Dünner Draht nach Anspruch 1, bei dem der Draht das beta-Teilchenemitterradioisotop in das Material des Drahtes eingearbeitet umfaßt.

3. Dünner Draht nach Anspruch 1, bei dem der Draht das beta-Teilchenemitterradioisotop auf der Oberfläche des Drahtes als Beschichtung umfaßt.

4. Dünner Draht nach einem der Ansprüche 1 bis 3, bei dem das beta-Teilchenradioisotop eine Halbwertszeit von weniger als 100 Tagen hat.

5. Dünner Draht nach Anspruch 4, bei dem das beta-Teilchenemitterradioisotop Vanadin 48 oder Gold 189 ist.

6. Dünner Draht nach einem der Ansprüche 1 bis 5, bei dem der Draht mit anti-thrombogenem Material beschichtet ist.

7. Dünner Draht nach einem der Ansprüche 1 bis 3, bei dem das beta-Teilchenemitterradioisotop Phosphor 32 ist.

## Revendications

1. Fil fin comprenant une extrémité radioactive pour utilisation à la prévention de la resténose d'une artère à la suite d'un trauma artériel, ledit fil fin, étant conformé de sorte que l'on peut insérer temporairement ladite extrémité radioactive en regard du site d'un trauma de paroi artérielle et ensuite la retirer, et sert, lorsqu'on l'a ainsi inséré à prévenir la resténose de l'artère en regard du site de trauma, ladite extrémité radioactive comprenant un radio-isotope émetteur de particules bêta et émettant des particules bêta.

2. Fil fin selon la revendication 1, dans lequel le fil comprend, incorporé dans la matière de fil, ledit radio-isotope émetteur de particules bêta.

3. Fil fin selon la revendication 1, dans lequel le fil comprend, plaqué sur la surface du fil, ledit radio-isotope émetteur de particules bêta.

4. Fil fin selon l'une quelconque des revendications 1 à 3, dans lequel ledit radio-isotope de particules bêta a une demi-vie de moins de 100 jours.

5. Fil fin selon la revendication 4, dans lequel ledit radio-isotope émetteur de particules bêta est du vanadium 48 ou de l'or 198.

6. Fil fin selon l'une quelconque des revendications 1 à 5, dans lequel ledit fil est recouvert d'une matière anti-thrombogénique.

7. Fil fin selon l'une quelconque des revendications 1 à 3, dans lequel ledit radio-isotope émetteur de particules bêta est du phosphore 32.
